# EUROPEAN PATENT APPLICATION

(11) **EP 4 549 955 A1**
(43) Date of publication of application: **07.05.2025**
(21) Application number: 23831815.8
(22) Date of filing: 23.06.2023
(51) Int. Cl.: G01N 35/10, G01N 35/00

(54) **AUTOMATED SAMPLE POOLING SYSTEM AND SAMPLE POOLING METHOD USING SAME**

(30) Priority: 28.06.2022 KR 20220078778
(71) Applicant: Seegene, Inc., Seoul 05548 (KR)
(72) Inventor: KIM, Seong Su, Uijeongbu-si Gyeonggi-do 11803 (KR); JOO, Jae Hyung, Yongin-si Gyeonggi-do 16843 (KR); CHOI, Jin Hwan, Namyangju-si Gyeonggi-do 12248 (KR); MIN, Eun Hye, Seongnam-si Gyeonggi-do 13260 (KR)
(74) Representative: Viering, Jentschura & Partner mbB Patent- und Rechtsanwälte
(86) International application number: PCT/KR2023/008733
(87) International publication number: WO 2024/005455

(57) **Abstract**

The present disclosure relates to a sample pooling method using an automatic sample pooling system, the sample pooling method including arranging a plurality of pooling chambers and a plurality of sample vessels, associating the sample vessels equal to a preset pool size with each of the pooling chambers, and transferring samples from the sample vessels to the associated pooling chambers by using sample transfer mechanisms, to provide a pooled sample in the pooling chamber where the samples equal to the pool size are combined.

## Description

### Technical Field

The present disclosure relates to an automated sample pooling system that may be used for detecting a nucleic acid reaction, and a sample pooling method using the sample pooling system.

### Background Art

Polymerase chain reaction (PCR) is the most widely used nucleic acid amplification method, encompassing repeated cycles of denaturation of double-stranded deoxyribonucleic acid (DNA), annealing of oligonucleotide primers to the DNA templates, and extension of the primers by DNA polymerase. Denaturation of DNA occurs at about 95 °C, and annealing and extension of a primer occur at a temperature lower than 95 °C, i.e., between 55 °C and 75 °C.

Traditional infectious disease diagnostic tests are performed by individually processing samples collected from respective individuals, and performing target analyte detection. However, this method requires the use of one kit per sample, which leads to high costs and increased time consumption. Moreover, when the positive rate is significantly low, most tests yield negative results, but individual testing for each sample is still required, leading to a waste of cost and time.

To overcome these challenges, a pooling testing approach is employed, in which a plurality of samples are mixed and tested as a single unit. However, such pooling testing methods rely on manual sample pooling by testers, and an automated process for sample pooling and testing has yet to be developed.

A manual pooling process involves manually assigning a bundle of samples to be pooled and manually inputting data associated with the samples, increasing the likelihood of errors in a pooling process or data entry.

Therefore, there is a need for an automated sample pooling system for automatically assigning samples to be pooled and performing a pooling process, and a sample pooling method using the sample pooling system.

### Disclosure of Invention

### Technical Problem

In the foregoing background, the present inventors have endeavored to develop a method for implementing an automatic sample pooling system by using a liquid handling device, and thereby automatically performing pooling.

As a result, the present inventors have developed a method by which an automatic sample pooling system automatically associates samples to be pooled, with a pooling container, and transfers the samples to the associated pooling container.

Thus, it is an objective of the present disclosure to provide a sample pooling method using an automatic sample pooling system.

It is another object of the present disclosure to provide an automatic sample pooling system capable of implementing the sample pooling method.

### Technical Solution

To achieve the foregoing objectives, an aspect of the disclosure may provide a sample pooling method using an automatic sample pooling system comprising a deck and a plurality of sample transfer mechanisms, the sample pooling method comprising: a pooling chamber arrangement step of arranging a plurality of pooling chambers in a pooling area of the deck; a sample vessel arrangement step of arranging a plurality of sample vessels in a sample area of the deck; a sample association step of associating the sample vessels equal to a preset pool size to each of the pooling chambers, wherein the pool size represents the number of sample vessels to be associated with each of the pooling chambers; and a sample pooling step of transferring samples from the sample vessels to the associated pooling chamber by using the sample transfer mechanisms, to provide a pooled sample in the pooling chamber where the samples equal to the pool size are combined.

The pool size may be selected within a range of 2 to 10.

In the sample association step, p samples may be associated with the pooling chamber, and positions in a processing order may be sequentially assigned to the sample vessels associated with the pooling chamber, and in the sample pooling step, a preset amount of the samples in the sample vessels may be transferred to the associated pooling chamber sequentially according to the processing ranks that are assigned to the sample vessels.

The sample pooling method may further comprise a task group grouping step of grouping the pooling chambers in the pooling area into pooling task groups, wherein each of the pooling task groups comprises the pooling chambers equal to a preset task group size, and the task group size represents the number of pooling chambers included in each of the pooling task groups, wherein, in the sample pooling step, a process of transferring a preset amount of the sample from each of the sample vessels corresponding to a same processing ranks among the sample vessels associated with each of the pooling chambers in the pooling task group, to each associated pooling chamber is sequentially performed.

In the pooling area, the plurality of pooling chambers may be arranged in an orthogonal array, and in the task group grouping step, the pooling chambers located in a same row or a same column may be grouped into a same pooling task group.

In the sample area, the plurality of sample vessels may be arranged in an orthogonal array, and in the task group grouping step, the sample vessels located in a same row or a same column among the sample vessels associated with the pooling chamber of the pooling task group may be grouped into a sample task group having a same processing order.

The plurality of sample transfer mechanisms may comprise the sample transfer mechanisms corresponding to the sample vessels within the sample task group, and in the sample pooling step, a preset amount of the samples from the sample vessels within the sample task group may be transferred to the respective pooling chambers associated with the sample vessels by using the sample transfer mechanisms.

The sample pooling method may further comprise, before the sample association step, a pool size input step of receiving, as input, the pool size, which is the number of sample vessels to be associated with each of the pooling chambers.

The sample pooling method may further comprise: a sample identifier generation step of generating sample identifiers for the samples accommodated in the sample vessels in the sample area, respectively; a pool identifier generation step of generating pool identifiers for the pooled samples accommodated in the pooling chambers in the pooling area, respectively; and a pool identifier mapping step of mapping the sample identifiers of the sample vessels associated with each of the pooling chambers, to the pool identifier.

The sample pooling method may further comprise an error alarm step of, when an error has occurred in a process of transferring the samples from the sample vessels associated with the pooling chamber, providing information about at least one of a location of the sample vessel for which the error has occurred, and a location of the pooling chamber associated with the sample vessel for which the error has occurred.

In the error alarm step, information about the location of the sample vessel for which the error has occurred may be provided by positioning an indicator on the sample vessel.

The sample transfer mechanism may comprise a detachable tip for sample aspiration, and in the error alarm step, the information about the location of the sample vessel for which the error has occurred may be provided by moving the sample transfer mechanism to be positioned above the sample vessel for which the error has occurred, and then separating the tip and positioning the tip within the sample vessel.

In the sample pooling step, the pooled sample may be transferred in a state in which the pooling chambers in the pooling area each contain a lysis buffer and/or magnetic beads.

The automatic sample pooling system may further comprise a sample lysis area in which a plurality of lysis vessels corresponding to the pooling chambers, respectively, and containing a lysis buffer are arranged, and the sample pooling method may further comprise, after the sample pooling step, a sample lysis step of transferring the pooled sample of each of the pooling chambers in the pooling area, to the corresponding lysis vessel.

To achieve the foregoing objectives, another aspect of the disclosure may provide a computer-readable recording medium comprising instructions for implementing a processor for executing a sample pooling method, the sample pooling method comprising: a sample association step of associating sample vessels equal to a preset pool size among a plurality of sample vessels in a sample area, to each of a plurality of pooling chambers in a pooling area, wherein the pool size represents the number of sample vessels to be associated with each of the pooling chambers; and a sample pooling step of transferring samples from the sample vessels to the associated pooling chamber, to provide a pooled sample in the pooling chamber where the samples equal to the pool size are combined.

To achieve the foregoing objectives, another aspect of the disclosure may provide an automatic sample pooling device comprising a computer processor and the computer-readable recording medium of claim 15, which is coupled to the computer processor.

To achieve the foregoing objectives, another aspect of the disclosure may provide a computer program that is stored in a computer-readable recording medium, and implements a processor for executing a sample pooling method, the sample pooling method comprising: a sample association step of associating sample vessels equal to a preset pool size among a plurality of sample vessels in a sample area, to each of a plurality of pooling chambers in a pooling area, wherein the pool size represents the number of sample vessels to be associated with each of the pooling chambers; and a sample pooling step of transferring samples from the sample vessels to the associated pooling chamber, to provide a pooled sample in the pooling chamber where the samples equal to the pool size are combined.

To achieve the foregoing objectives, another aspect of the disclosure may provide an automatic sample pooling system comprising: a deck comprising a sample area in which a plurality of sample vessels are arranged, and a pooling area in which a plurality of pooling chambers are arranged; and a plurality of sample transfer mechanisms configured to transfer samples from the sample vessels to the pooling chambers, wherein the number of sample vessels in the sample area is determined by multiplying the number of pooling chambers in the pooling area by a pool size that is selected within a range of 2 to 10.

The automatic sample pooling system may further comprise a controller configured to associate the sample vessels equal to the pool size, with each of the pooling chambers in the pooling area, and control the sample transfer mechanisms to transfer samples in the sample vessels to the associated pooling chamber, to provide a pooled sample in the pooling chamber where the samples equal to the pool size are combined.

The automatic sample pooling system may further comprise a controller configured to receive information about the pool size, associate the sample vessels equal to the pool size, with each of the pooling chambers in the pooling area, sequentially assign positions in a processing order to the sample vessels associated with the pooling chamber, and control the sample transfer mechanisms to transfer samples in the sample vessels to the associated pooling chamber according to the assigned processing ranks, to provide a pooled sample in the pooling chamber where the samples equal to the pool size are combined.

The controller may be further configured to generate sample identifiers for the samples accommodated in the sample vessels in the sample area, respectively, generate pool identifiers for the pooled samples accommodated in the pooling chambers in the pooling area, respectively, and associate the sample identifiers of the sample vessels associated with each of the pooling chambers, to the pool identifier.

The controller may be further configured to, when an error has occurred during a process of transferring the samples from the sample vessels associated with the pooling chamber, receive an error signal from the sample transfer mechanism and provide information about at least one of a location of the sample vessel for which the error has occurred and a location of the pooling chamber associated with the sample vessel for which the error has occurred.

The controller may be further configured to output an instruction to position an indicator on the sample vessel for which the error has occurred, to provide the information about the location of the sample vessel for which the error has occurred.

The controller may be further configured to output an instruction to display the information about the location of the pooling chamber associated with the sample vessel for which the error has occurred, to provide the information about the location of the pooling chamber associated with the sample vessel for which the error has occurred.

The pooling chambers in the pooling area each may contain a lysis buffer and/or magnetic beads.

The automatic sample pooling system may further comprise a sample lysis area in which a plurality of lysis vessels corresponding to the pooling chambers, respectively, and containing a lysis buffer and/or magnetic beads are arranged.

The pooling area may be configured to have mounted thereon the pooling chambers arranged in 8 rows and 12 columns.

### Advantageous Effects of Invention

According to a method of the present disclosure, errors in sample pooling may be reduced.

In detail, it is possible to prevent a problem in which information about a plurality of specimen samples associated with a pooled sample does not match the actual specimen samples mixed in the pooled sample due to a dispensing error of mixing a specimen sample other than a pooling target specimen sample or an information allocation error of incorrectly inputting information about specimen samples associated with a pooled sample.

In addition, a system of the present disclosure has an advantage in that it includes a plurality of sample transfer mechanisms and thus is able to obtain a plurality of pooled samples simultaneously.

In addition, the system of the present disclosure includes an indicator for indicating an error, and thus is able to, when an error has occurred while a sample transfer mechanism aspirates a sample, intuitively recognize the occurrence of the error and subsequently cope with the error, by using the indicator.

In addition, the system of the present disclosure performs sample aspiration, starting from a sample in a sample vessel closer to a sample pooling container, thereby preventing a sample from being dropped into other sample vessels before sample pooling while the sample transfer mechanism is moving.

### Brief Description of Drawings

FIG. 1 is a diagram illustrating a sample pooling system according to an embodiment of the present disclosure.
FIGS. 2 to 4 are plan views illustrating a sample mounting portion.
FIG. 5 is a plan view illustrating pooling chambers in a pooling area.
FIG. 6 is a flowchart of a sample pooling method according to an embodiment of the present disclosure.
FIG. 7 is a flowchart of a sample pooling method according to another embodiment of the present disclosure.

### Mode for the Invention

Hereinafter, the present disclosure will be described in more detail with reference to embodiments. The embodiments are for illustrative purposes only, and it should be apparent to those of skill in the art that the scope of the present disclosure is not limited to the embodiments.

In addition, in describing components of the present disclosure, expressions such as 'first', 'second', 'A', 'B', '(a)', or '(b)' may be used. These expressions are only intended to distinguish one component from another, and do not limit the nature, order, or sequence of the components. It should be understood that, when it is described that a first element is "connected," "coupled," or "joined" to a second element, the first element may be directly connected, coupled, or joined to the second element, or the first element may be connected, coupled, or joined to the second element with a third element connected, coupled, or joined therebetween.

### I. Automatic sample pooling system

According to an embodiment of the present disclosure, provided is an automatic sample pooling system including: a deck including a pooling area in which a sample pooling container including a plurality of pooling chambers is arranged, and a sample area in which a plurality of sample vessels are arranged, the number of which is 2 to 10 times the number of the plurality of pooling chambers of the sample pooling container; and a plurality of sample transfer mechanisms configured to transfer samples to the plurality of pooling chambers.

As used herein, the term "sample" may include a biological sample (e.g., cells, tissues, and fluids from a biological source) and a non-biological sample (e.g., food, water and soil). Examples of the biological sample may include viruses, bacteria, tissues, cells, blood (e.g., whole blood, plasma, or serum), lymph, bone marrow fluid, saliva, sputum, swab, aspiration, milk, urine, feces, ocular fluid, semen, brain extract, spinal fluid, joint fluid, thymic fluid, bronchoalveolar lavage fluid, ascites, and amniotic fluid. In addition, the sample may include natural nucleic acid molecules isolated from a biological source, and synthetic nucleic acid molecules. According to an embodiment of the present disclosure, the sample may include an additional substance such as water, deionized water, saline solution, pH buffer, acid solution, or alkaline solution.

In addition, as used herein, the term "sample" may include a specimen collected from a patient. In addition, as used herein, the term "pooled sample" may refer to a state in which samples collected from different patients are mixed.

In addition, as used herein, the term "sample" may also refer to a primary pooled sample in which samples collected from different patients are mixed. In this case, the term "pooled sample" may refer to a secondary pooled sample in which different primary pooled samples are mixed.

In addition, the sample vessel is used to accommodate a sample to be analyzed, and includes various types of vessels, for example, a tube, a vial, a strip having a plurality of tubes connected thereto, a plate having a plurality of tubes connected thereto, a microcard, a chip, a cuvette, or a cartridge.

Pooling testing refers to a method in which samples from multiple individuals are mixed into a single sample for testing, and when the sample tests positive, the remaining individual samples are retested separately. According to currently known pooling testing methods, even when mixing 10 samples for testing, it is known that sensitivity of 96 % or higher may be maintained compared to individual sample testing.

Sample pooling refers to a process of mixing a plurality of samples for pooling testing. According to an embodiment of the present invention, an automatic sample pooling system may combine or mix multiple samples in a single pooling chamber by using a sample transfer mechanism, and associate sample vessels to pooling chambers, enabling tracking of an individual sample that tests positive.

In the present specification, the terms "specimen" and "sample" may be used interchangeably in some cases, while in other cases they may have distinct meanings. The term "sample" may refer to a specimen sample collected from a patient, and in a case of performing secondary pooling, it may also include a pooled sample obtained by pooling specimen samples. That is, in the present specification, the term "sample" is used in a broad sense to refer to an object to be pooled, and may refer to a specimen sample or a product of primary pooling to be subjected to secondary pooling.

In addition, in the present specification, the terms "sample" and "sample vessel" may be used interchangeably in some cases, while in other cases they may have distinct meanings.

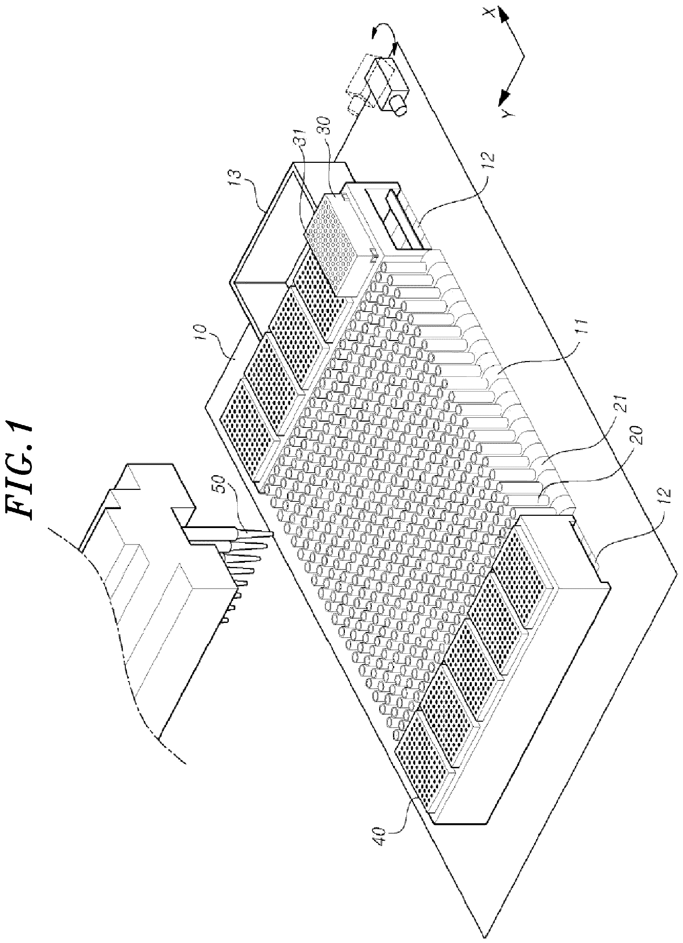

FIG. 1 illustrates an automatic sample pooling system according to an embodiment of the present disclosure.

The automatic sample pooling system may include a deck 10, and the deck 10 may include a pooling area in which pooling chambers are arranged, a sample area in which sample vessels are arranged, and a tip area in which replaceable tips are arranged.

In addition, the deck 10 may be equipped with a sample carrier 21 that accommodates sample vessels 20, a sample pooling container 30, and a tip container 40 that accommodates a plurality of tips.

The deck 10 may include a sample mounting portion 11 on which the sample carriers 21 are mounted, and container mounting portions 12 on which the tip container 40 or the sample pooling container 30 may be mounted. Referring to FIG. 1, the sample mounting portion is located in a central area of the deck 10, and the container mounting portions 12 may be located on the left and right sides of the deck 10, respectively.

The sample mounting portion 11 may have mounted thereon a row of sample carriers 21 arranged in parallel in the x-direction, each of which accommodates a plurality of sample vessels 20 arranged in the y-direction. In addition, the sample mounting portion 11 may have mounted thereon sample carriers 21 on which sample vessels 20 of various sizes may be mounted.

The sample carrier 21 may be provided in a modular form to hold sample vessels 20 of different sizes and be mounted on the sample mounting portion 11. For example, the sample mounting portion 11 may have mounted thereon a sample carrier in which 24 sample vessels with a diameter of 16 mm are arranged in the y-direction, or a sample carrier in which 32 sample vessels with a diameter of 12 mm are arranged in the y-direction.

In addition, the sample mounting portion 11 may have mounted thereon a total of 192 or more sample vessels 20. The sample mounting portion 11 may have mounted thereon more sample vessels 20, for example, a total of 288 or more sample vessels 20. The sample mounting portion 11 may have mounted thereon more sample vessels 20, for example, a total of 384 or more sample vessels 20. The sample mounting portion 11 may have mounted thereon more sample vessels 20, for example, a total of 480 or more sample vessels 20. For example, the sample mounting portion 11 may have mounted thereon 24 or more sample carriers each accommodating sample vessels with a diameter of 16 mm, and may have mounted thereon 15 or more sample carriers each accommodating sample vessels with a diameter of 12 mm.

The sample vessel may include a barcode. The barcode may be used to track matching data between a specimen donor and the sample vessel. In addition, the automatic sample pooling system may include a barcode reader 14 capable of reading a barcode of a sample vessel.

In addition, a plurality of sample carriers 21 may be bound together as a single unit by a sample carrier fixing jig (not shown). For example, when five samples are mixed in a pooled sample, five sample carriers 21 may be bound together as a single unit by the fixing jig. The sample carrier 21 may be mounted on the deck 10 while fixed to the fixing jig, and may be separated from the fixing jig while being mounted on the deck 10.

The container mounting portions 12 may be provided such that four containers may be mounted in the y-direction on the left side of the deck 10, and four containers may be mounted in the y-direction on the right side of the deck 10.

The tip container 40 and the sample pooling container 30 may be provided in a modular form to be mounted on the container mounting portion 12. For example, the left container mounting portion 12 may have mounted thereon four tip containers 40, and the right container mounting portion 12 may have mounted thereon three tip containers 40 and one sample pooling container 30.

The tip container 40 may have mounted thereon a total of 96 tips in an 8-row x 12-column configuration. In addition, the sample pooling container 30 may be provided with a total of 96 pooling chambers 31 arranged in an 8 row x 12 column configuration. For example, the sample pooling container 30 may be a 96-well plate, and the pooling chambers 31 may correspond to 96 wells.

In the deck 10, the sizes and locations of the pooling area in which the sample pooling container 30 is mounted, and the tip area in which the tip container 40 is mounted may be variously adjusted. In addition, the sizes and shapes of the sample carrier 21, the sample pooling container 30, and the tip container 40 may also be variously adjusted.

The number of samples or specimens to be mixed in a pooled sample may be selected within the range of 2 to 10. While it is possible to pool more than 10 samples (e.g., 16, 20, 32, or 48 samples) in some cases, pooling 10 or fewer samples is frequently performed to ensure a sensitivity of 96 % or higher compared to individual sample testing. Alternatively, to ensure a pooling sensitivity of 98 % or higher while reducing the total number of tests, the number of samples mixed in a pooled sample may be selected within the range of 3 to 5.

In addition, the automatic sample pooling system may include a sample transfer mechanism 50. The sample transfer mechanism 50 may aspirate a sample from the sample vessel 20 and then move to dispense the sample into the pooling chamber 31 of the sample pooling container 30. For example, a pipette capable of aspirating and dispensing a quantitative amount of fluid may be used for the sample transfer mechanism 50.

In addition, the sample transfer mechanism 50 may be provided to be movable in the x-direction and the y-direction. In addition, the sample transfer mechanism 50 may be provided to be movable in the up and down directions. For example, the sample transfer mechanism 50 may move to the tip container 40 of the container mounting portion 12 on the left side of the sample mounting portion 11 to attach a tip, then move to the sample mounting portion 11 to aspirate a sample from the sample vessel 20, and then move to the sample pooling container 30 of the container mounting portion 12 on the right side of the sample mounting portion 11 to dispense the sample into the pooling chamber 31. In addition, the sample transfer mechanism 50 may move to a waste container to discard the used tip.

In addition, the automatic sample pooling system may include a plurality of sample transfer mechanisms 50 to simultaneously aspirate a plurality of samples and transfer the samples to the pooling chambers 31 of a plurality of sample pooling containers 30. In this case, the plurality of sample transfer mechanisms 50 may be connected to a robotic arm. For example, the plurality of sample transfer mechanisms 50 may be arranged in parallel in the y-direction of the deck 10, and 8 sample transfer mechanisms 50 may be arranged in parallel in the y-direction.

In addition, the sample transfer mechanisms 50 may move independently of each other. For example, the plurality of sample transfer mechanisms 50 may move independently in the y-direction of the deck 10, and the spacing between adjacent sample transfer mechanisms 50 may be adjusted. For example, in a case in which 12-mm sample vessels 20 are mounted on the sample carrier 21, the sample transfer mechanisms 50 may move in the y-direction to reduce the spacings between them, and in a case in which 16-mm sample vessels 20 are mounted on the sample carrier 21, the sample transfer mechanisms 50 may move in the y-direction to increase the spacings between them.

In addition, the tip container (40) may have a plurality of tips mounted thereon. The plurality of tips may be arranged in an orthogonal array in the x-direction and the y-direction of the deck 10. The sample transfer mechanism 50 may retrieve and attach one tip from the plurality of tips in the tip container 40, and then move to one of the plurality of sample vessels 20 to aspirate a sample. Here, more tips may be provided than the number of sample vessels 20 containing samples.

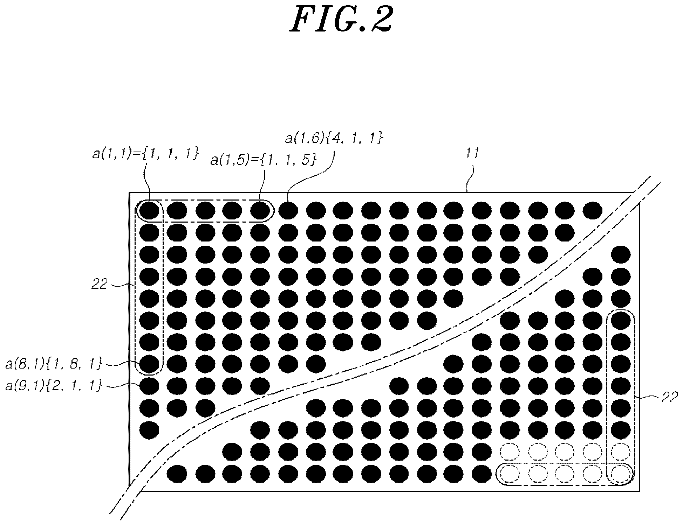

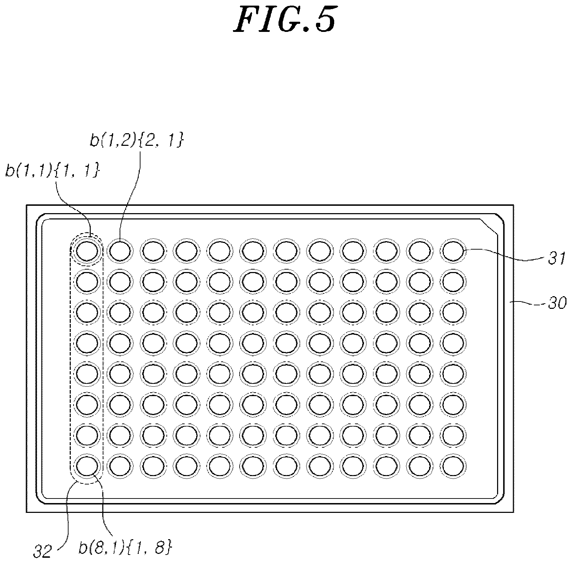

FIGS. 2 to 4 are plan views illustrating a sample mounting portion, and FIG. 5 is a plan view illustrating pooling chambers in a pooling area.

The sample mounting portion 11 may include a plurality of sample vessels 20. The plurality of sample vessels 20 may be arranged in an orthogonal array in the x-direction and the y-direction of the sample mounting portion 11. Referring to FIG. 2, a total of 480 sample vessels 20 may be provided, including 24 sample vessels 20 arranged in the y-direction of the sample mounting portion 11, and 20 sample vessels 20 arranged in the x-direction.

In addition, the sample pooling container 30 may include a plurality of pooling chambers 31. The plurality of pooling chambers 31 may be arranged in an orthogonal array in the x-direction and the y-direction of the deck 10. Referring to FIG. 5, a total of 96 pooling chambers 31 may be provided, including 8 pooling chambers 31 arranged in the y-direction of the sample pooling container 30, and 12 pooling chambers 31 arranged in the x-direction.

The pooling chamber 31 may be provided in the form of a well that is recessed from the upper surface of the sample pooling container 30. For example, the sample pooling container 30 may be a well plate, and may be a 96-well plate with 96 wells arranged in 8 rows and 12 columns (an 8-row x 12-column configuration).

The sample pooling container 30 may include a barcode. The barcode may be used to track matching data between a pooled sample and the sample pooling container 30. In addition, the barcode reader 14 of the automatic sample pooling system may read the barcode of the sample pooling container 30.

In addition, the barcode reader 14 may be provided to be rotatable. The barcode reader 14 may read the barcodes of a plurality of sample vessels 20 at a first position during a process of mounting the sample carrier 21, and may read the barcode of the sample pooling container 30 at a second position after rotating by a certain angle.

In addition, the automatic sample pooling system may include a controller. The controller may associate sample vessels 20 with each of the plurality of pooling chambers 31, and output an instruction for driving the sample transfer mechanism 50 to transfer samples accommodated in the sample vessels 20 to the associated pooling chamber 31.

Here, the process of associating the plurality of sample vessels 20 with the pooling chambers 31 may also be understood as a matching process or an assignment process.

In addition, the controller may receive information about the number of samples to be associated to each pooling chamber 31, and associate p samples to each pooling chamber 31 of the sample pooling container 30. Here, p may be expressed as a pool size.

In addition, the controller may assign, to the sample vessels 20 associated with each pooling chamber 31, a position in a processing order, from the first position to the p-th position. In addition, the controller may output an instruction signal to transfer a sample in the associated sample vessels 20 according to their assigned processing ranks, to each pooling chamber 31.

In addition, the controller may group the plurality of pooling chambers 31 into two or more pooling task groups 32. Each pooling task group 32 may include pooling chambers 31 equal to a preset task group size. Here, the pooling task groups 32 may be provided with the same task group size.

In addition, the controller may group the plurality of sample vessels 20 into two or more sample task groups 22. Each sample task group 22 may include sample vessels 20 equal to a preset task group size. Here, the sample task groups 22 may be provided with the same task group size.

In addition, the pooling task groups 32 and the sample task groups 22 may be provided with the same task group size.

Hereinafter, with reference to FIGS. 2 to 5, an embodiment will be described, in which the sample vessels 20 are arranged in an orthogonal array of a 24-row x 20-column configuration, and the pooling chambers 31 are arranged in an orthogonal array of an 8-row x 12-column configuration.

When a user inputs, to an input unit (not shown), '5' as a pool size to be executed, the controller may associate 5 samples to each pooling chamber 31. In addition, the sample vessels 20 in the same row may be assigned with positions in a processing order, sequentially in the x-direction.

In addition, the task group sizes of the pooling task groups 32 and the sample task groups 22 may be set to be equal to the number of rows of the pooling chambers 31, and the drawings illustrate that the task group sizes are set to 8.

In addition, the controller may generate a sample identifier for a sample accommodated in each of the plurality of sample vessels 20 in the sample mounting portion 11. The sample identifier may include matching data on barcode information of the sample vessel 20 and location information of the sample vessel 20.

In addition, the controller may generate a pool identifier for a pooled sample accommodated in each of the plurality of pooling chambers 31 in the sample pooling container 30. The pool identifier may include matching data on barcode information of the sample pooling container 30 and location information of the pooling chamber 31.

In addition, the controller may map a plurality of sample identifiers to each pool identifier.

In addition, the controller may store, in a storage unit, the barcode information of the sample vessel 20, the sample identifier, the pool identifier, the barcode information of the sample pooling container 30, and mapping information therebetween.

Hereinafter, a process of mapping sample identifiers to pool identifiers will be described with reference to FIGS. 2 to 5. However, the following examples describe some of various possible embodiments, and the present disclosure may include various other embodiments.

A sample identifier may include location information '(row (r), column (c))' and task information '{task group (g), sample number in task group (n), position in processing order (p)}', and for example, the location information and the task information may be matched to be expressed as 'a(r,c)={g,n,p}'. The location information is provided for user convenience and may not be generated if necessary.

For example, a sample located in the first row and the first column may be expressed as 'a(1,1)={1,1,1}', and a sample located in the first row and the fifth column may be expressed as 'a(1,5)={1,1,5}'. In addition, a sample located in the eighth row and the first column may be expressed as 'a(8,1)={1,8,1}', and a sample located in the eighth row and the fifth column may be expressed as 'a(8,5)={1,8,5}'. In addition, a sample located in the ninth row and the first column may be expressed as 'a(9,1)={2,1,1}', and a sample located in the first row and the sixth column may be expressed as 'a(1,6)={4,1,1}'.

In addition, a pool identifier may include location information '(row (r), column (c))' and task information '{task group (g), sample number in task group (n)}', and for example, may be expressed as 'b(r,c)={g,n}'. The location information is provided for user convenience and may not be generated if necessary.

For example, a pooled sample located in the first row and the first column may be expressed as 'b(1,1)={1,1}', and a pooled sample located in the first row and the second column may be expressed as 'b(1,2)={2,1}'. In addition, a pooled sample located in the eighth row and the first column may be expressed as 'b(8,1)={1,8}', and a pooled sample located in the eighth row and the second column may be expressed as 'b(8,2)={2,8}'.

In addition, the controller may associate the samples (or the sample vessels 20) to the pooling chamber 31 according to the order of the task groups (g).

A process of associating the first sample task group 22 with the first pooling task group 32 is as follows. The controller may associate the sample vessels 20 at 'a(1,1)' to 'a(1,5)' with the pooling chamber 31 at 'b(1,1)', and associate the samples at 'a(2,1)' to 'a(2,5)' with the pooling chamber 31 at 'b(2,1)'. After repeating these processes, the sample vessels 20 at 'a(8,1)' to 'a(8,5)' may be associated with the pooling chamber 31 at 'b(8,1)'.

A process of associating the second sample task group 22 with the second pooling task group 32 is as follows. The controller may associate the sample vessels 20 at 'a(9,1)' to 'a(9,5)' with the pooling chamber 31 at 'b(1,2)', and associate the samples at 'a(10,1)' to 'a(10,5)' with the pooling chamber 31 at 'b(2,2)'.

In addition, referring to FIG. 2, sample pooling may start from the sample vessel 20 accommodating the sample at 'a(1,1)', which is located at the upper left corner of the sample mounting portion 11. In this case, the processing ranks (p) may be set sequentially in the rightward direction, and the sample numbers (n) in the same sample task group 22 may be set sequentially in the downward direction.

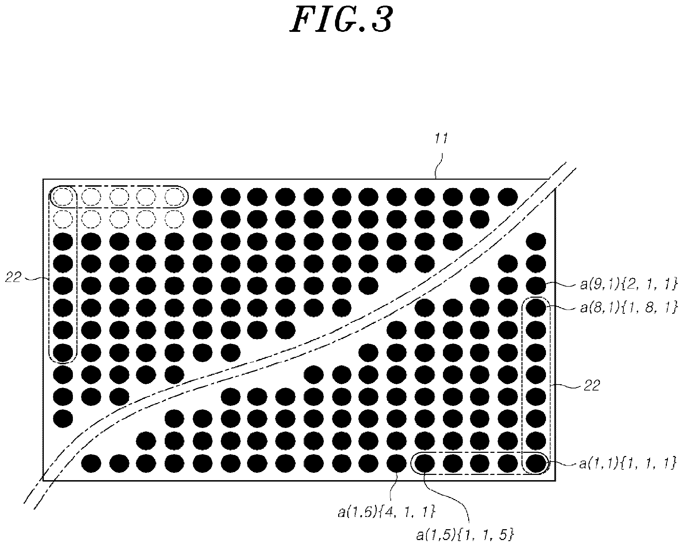

Alternatively, referring to FIG. 3, sample pooling may start from the sample vessel 20 accommodating the sample at 'a(1,1)', which is located at the lower right corner of the sample mounting portion 11. In this case, the processing ranks (p) may be set sequentially in the leftward direction, and the sample number (n) in the same sample task group 22 may be set sequentially in the upward direction.

In this way, the system according to the present disclosure performs sample aspiration, starting from a sample in the sample vessel 20 closer to the sample pooling container 30, thereby preventing a sample from being dropped into other sample vessels 20 before sample pooling while the sample transfer mechanism 50 is moving. In detail, the path along which the sample transfer mechanism 50 aspirates a sample and then moves to the sample pooling container 30 may pass over the sample vessels 20 from which samples have been aspirated.

In addition, the sample mounting portion 11 may accommodate a total of 480 sample vessels 20 such that a total of 96 pooled samples are obtained, but there is a need to leave two pooling chambers 31 empty for a negative control (NC) and a positive control (PC) in a sample preparation step after sample pooling. That is, in the sample mounting portion 11, a total of 94 pooled samples may be obtained by using a total of 470 samples. In this case, in each of the last five sample carriers 21, two slots may be empty without sample vessels 20.

For example, referring to FIG. 2, when pooling starts from the upper left corner of the sample mounting portion 11, the five sample carriers located on the rightmost side of the x-axis may each have two empty slots without sample vessels from the lower side of the y-axis.

Alternatively, referring to FIG. 3, when pooling starts from the lower right corner of the sample mounting portion, the five sample carriers located on the leftmost side of the x-axis may each have two empty slots without sample vessels from the upper side of the y-axis.

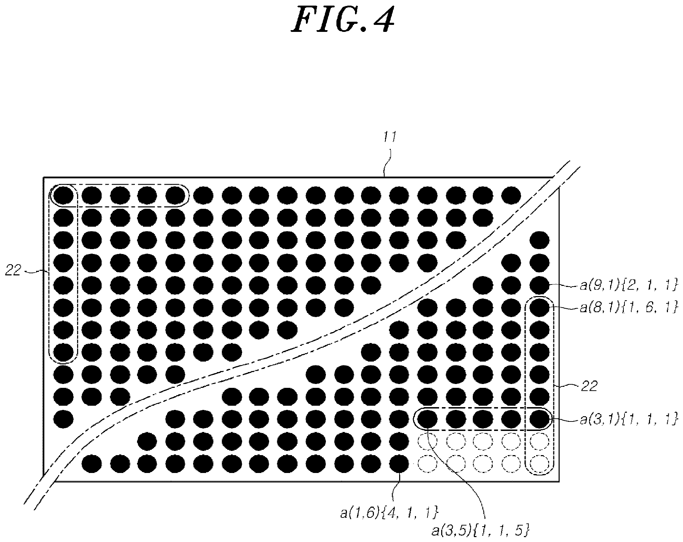

Alternatively, referring to FIG. 4, even when pooling starts from the lower right corner of the sample mounting portion, the five sample carriers located on the rightmost side of the x-axis may each have two empty slots without sample vessels from the lower side of the y-axis. In this case, the first sample is accommodated in the sample vessel at 'a(3,1)', which is the third sample vessel from the lower side of the y-axis in the sample carrier located on the rightmost side of the x-axis. This is to allow the user to intuitively recognize the number of empty sample vessels, because humans generally recognize the order of progress from the top left.

Thereafter, when polymerase chain reaction (PCR) has been performed on pooled samples accommodated in the sample pooling containers 30 after the sample preparation step, and some pooled samples have tested positive, it is possible to track in which pooling chambers 31 of which sample pooling containers 30 the pooled samples that have test positive are accommodated, through the barcodes of the corresponding containers and pool identifiers associated with or matched to the containers. In addition, the p samples mixed in the corresponding pooling chamber 31 may be identified by tracking the sample identifiers mapped to the pool identifier.

Thus, it is possible to identify which sample shows a positive result by performing PCR again on each of the p mapped samples, and to identify the patient who is determined to be positive by tracking mapping information between the sample identifier and the patient.

In addition, when an error has occurred in a process in which the sample transfer mechanism 50 transfers, to the pooling chamber 31, the associated sample vessels 20, the controller may receive an error signal from the sample transfer mechanism 50, and store and provide information about at least one of the location of the sample vessel 20 for which the error has occurred, or the location of the associated pooling chamber 31 for which the error has occurred. In this case, the error may be an error that occurs in a process in which the sample transfer mechanism aspirates a sample. For example, a clot error may occur, which refers to an error in which the inlet of the tip is blocked by a foreign substance and thus normal aspiration is not possible. The term "clot error" typically refers to an error that occurs during an aspiration process due to a blood clot formed while handling blood samples, but in the present specification, it refers comprehensively to cases in which the inlet of the tip is blocked by a foreign substance.

Then, the controller may output an instruction to replace the tip attached to the sample transfer mechanism, aspirate again the sample from the sample vessel 20 for which the error has occurred, and move the sample transfer mechanism to the associated pooling chamber 31.

Furthermore, the controller may output an instruction to position an indicator at the location of the sample for which the error has occurred, so as to provide information about the location of the sample vessel 20 for which the error has occurred. The indicator may be a separately provided member, or may be the tip attached to the sample transfer mechanism 50.

For example, when an error has occurred while aspirating a sample from the sample vessel 20, the tip may be separated from the sample transfer mechanism 50 and then positioned within the sample vessel 20. Then, an operator may recognize that the sample vessel 20 with the tip is the one for which the error has occurred, and transfer the sample to the target pooling chamber 31 through manual pipetting.

Alternatively, the controller may output an instruction to display information about at least one of the location of the sample vessel 20 for which the error has occurred or the location of the pooling chamber 31 with which the sample vessel 20 is associated.

In addition, the automatic sample pooling system may further include another sample pooling container 30 including a lysis buffer.

In an embodiment, the sample pooling container 30 may have a lysis buffer accommodated in the pooling chambers 31 therein. Alternatively, the pooling chambers 31 of the sample pooling container 30 may be empty, such that pooled samples may be transferred to the pooling chambers 31, and then the lysis buffer may be injected into the pooling chambers 31 in a later step.

In a case in which PCR requires 200 uL of a pooled sample, 200 uL of samples, which is a quantitative amount, needs to be dispensed into the pooling chamber 31 of the sample pooling container 30 including the lysis buffer.

The sample transfer mechanism 50 aspirates 40 uL of a sample from each of five sample vessels 20 and transfers the samples to the mapped pooling chamber 31 of the sample pooling container 30, and a total of 200 uL of a pooled sample, in which five samples are mixed, is accommodated in each pooling chamber 31.

In this case, a separate space in the deck 10 for accommodating a sample pooling container 30 including a lysis buffer is not required, and an additional process of transferring a pooled sample to the sample pooling container 30 including the lysis buffer is not required. In addition, because microelectronic pipettes capable of aspirating in units of 0.01 uL to 0.1 uL are commercially available, it is possible to dispense a quantitative amount of sample.

In another embodiment, the sample pooling container 30 may include a first sample pooling container 30 including empty pooling chambers 31, and a second sample pooling container 30 including pooling chambers 31 having a lysis buffer accommodated therein.

In a case in which PCR requires 200 uL of a pooled sample, 200 uL of samples, which is a quantitative amount, needs to be dispensed into the pooling chamber 31 of the second sample pooling container 30 including the lysis buffer.

In addition, the automatic sample pooling system may further include a sample pooling container including magnetic beads. For example, the sample pooling container including the magnetic beads may be provided separately from the sample pooling container 30 including the lysis buffer. Alternatively, the pooling chambers 31 of the sample pooling container 30 including the lysis buffer may be provided with both the lysis buffer solution and magnetic beads.

In the present specification, the term "magnetic beads" refer to particles or beads that react to a magnetic field. In general, the term "magnetic beads" refers to a material that does not have a magnetic field but forms a magnetic dipole that is exposed to a magnetic field. For example, it refers to a material that may be magnetized under a magnetic field, but does not have magnetism itself in the absence of a magnetic field. As used herein, the term "magnetism" includes both paramagnetic and superparamagnetic materials, but is not limited thereto.

For the purposes of the present disclosure, it is preferable that the magnetic beads have a property of binding to nucleic acids, and for example, may have a functional group that binds to nucleic acids, such as a -COOH group, but the present disclosure is not limited thereto.

The sample transfer mechanism 50 aspirates 80 uL of a sample from each of five sample vessels 20 and transfers the samples to the mapped pooling chamber 31 of the first sample pooling container 30, and a total of 400 uL of a pooled sample, in which five samples are mixed, is accommodated in each pooling chamber 31. In addition, the sample transfer mechanism 50 aspirates 200 uL of the pooled sample from each pooling chamber 31 of the first sample pooling container 30 and transfers the pooled sample to the pooling chamber 31 of the mapped second sample pooling container.

In this case, a separate space in the deck 10 for accommodating the second sample pooling container 30 is required, and an additional process of transferring a pooled sample from the first sample pooling container 30 to the second sample pooling container 30 is required. However, because only 200 uL of the pooled sample is aspirated from the pooling chamber 31 of the first sample pooling container 30, it is possible to dispense a quantitative amount of sample. In addition, when an additional pooled sample is required due to an error occurring in a PCR process, retesting is possible because 200 uL of an extra pooled sample remains in the first sample pooling container 30.

In addition, the automatic sample pooling system may further include a waste container 13 for storing waste such as used tips, and the barcode reader 14 capable of identifying a pool identifier and a sample identifier.

The sample transfer mechanism 50 may attach a tip from the tip container 40, transfer a sample from the sample vessel 20 to the sample pooling container 30, then discard the used tip into the waste container 13, and attach a new tip from the tip container 40 again.

In addition, when the sample carrier 21 is being mounted, the barcode reader 14 may identify a sample identifier (e.g., a barcode) of the sample vessel 20 mounted on the sample carrier 21. In some cases, the barcode reader 14 may also identify a sample carrier identifier that may identify the sample carrier 21.

In addition, the barcode reader 14 may identify a pool identifier (e.g., a barcode) of the sample pooling container 30. However, the controller may combine an identifier of the sample pooling container 30 with location information of each pooling chamber 31, and store and recognize the combination as a pool identifier.

In addition, the barcode reader 14 may be provided to be rotatable. The barcode reader may be provided to be rotatable, so as to recognize even a sample identifier and a pool identifier which are located at different locations and different heights.

### II. Sample pooling method

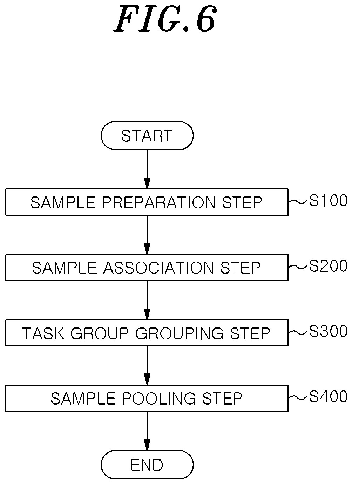

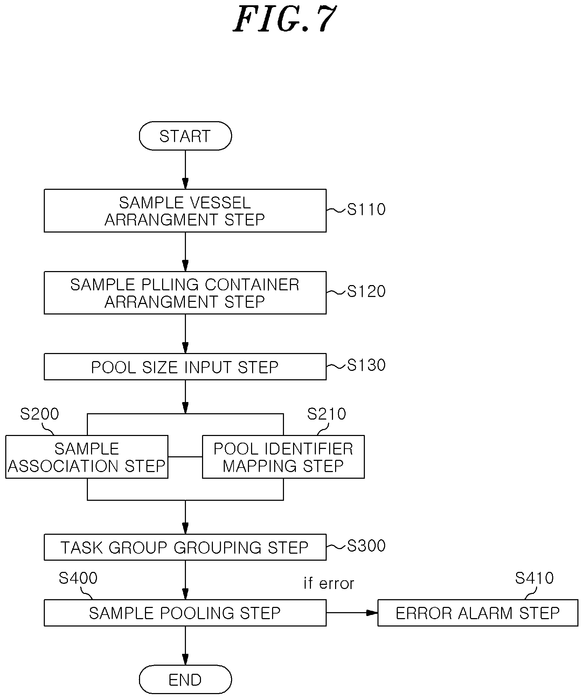

FIG. 6 is a flowchart of a sample pooling method according to an embodiment of the present disclosure, and FIG. 7 is a flowchart of a sample pooling method according to another embodiment of the present disclosure.

Referring to FIG. 6, the automatic sample pooling system may perform a sample preparation step S100, a sample association step S200, a task group grouping step S300, and a sample pooling step S400.

Referring to FIG. 7, the sample preparation step S100 may include a sample vessel arrangement step S110 of arranging the sample vessels 20 in the sample area, and a sample pooling container arrangement step S120 of arranging the sample pooling container 30 in the pooling area.

The sample vessel arrangement step S110 may be performed by mounting the sample vessels 20 on the sample carrier 21 and mounting the sample carrier 21 on the sample mounting portion 11 of the deck 10, which may be performed manually by an operator.

In addition, the sample pooling container arrangement step S120 may be performed by mounting the sample pooling container 30 on the container mounting portion 12, which may be performed manually by the operator.

In addition, the automatic sample pooling system may further include a pool size input step S130 of receiving, as input, the number of sample vessels 20 associated with each pooling chamber 31 (i.e., a pool size) before the sample association step S200. For example, the user may input a pool size within a range of 2 to 10 into an input means (e.g., a physical button or a touch button), and preferably, may input a pool size within a range of 3 to 5. Here, the pool size is an integer.

The sample association step S200 is a step of associating a plurality of sample vessels 20 with each pooling chamber 31 of the sample pooling container 30 including the plurality of pooling chambers 31 (receiving portions), and associating the same number of sample vessels 20 with each pooling chamber 31.

In the sample association step S200, p sample vessels 20 may be associated with the pooling chamber 31, and positions in a processing order, from the first position to the p-th position, may be assigned to the sample vessels 20 associated with the pooling chamber 31. For example, five sample vessels 20 arranged to be adjacent to each other in the x-direction in the sample area may be associated with one pooling chamber 31, and positions in a processing order may be assigned to the sample vessels 20, sequentially from left to right or from right to left.

In addition, the sample pooling step S400 is a step of transferring samples from the sample vessels 20 to the associated pooling chamber 31 to provide a pooled sample.

In the sample pooling step S400, the samples may be transferred to the associated pooling chamber 31 according to the processing ranks assigned to the sample vessels 20. For example, the sample transfer mechanism 50 may aspirate the sample from the sample vessel 20 with the first processing rank and dispense the sample into the associated pooling chamber 31. And after repeating this operation, the sample transfer mechanism 50 may aspirate the sample from the sample vessel 20 with the fifth processing rank and dispense the sample into the associated pooling chamber 31, such that five samples may be transferred to each pooling chamber 31.

In addition, the automatic sample pooling system may further include the task group grouping step S300 of grouping the plurality of pooling chambers 31 into two or more pooling task groups 32, wherein each pooling task group 32 includes the pooling chambers 31 equal to a preset task group size.

In the sample pooling step S400 after the task group grouping step S300, a step of dispensing a certain amount of sample from each of the sample vessels 20 with the same processing ranks among the sample vessels 20 associated with the respective pooling chambers 31 of the pooling task group 32, into each associated pooling chamber 31, may be performed sequentially from the samples with the first processing ranks to the samples with the p-th processing ranks. For example, eight sample transfer mechanisms 50 arranged in the y-direction to be adjacent to each other may aspirate samples from eight sample vessels 20 arranged in the y-direction with the first processing ranks and dispense the samples into eight associated pooling chambers 31, respectively. And after repeating this operation, the eight sample transfer mechanisms 50 may aspirate samples from eight sample vessels 20 with the fifth processing ranks and dispense the samples into eight associated pooling chambers 31, respectively.

In addition, in the task group grouping step S300, the pooling chambers 31 located in the same row or the same column may be grouped into the same pooling task group 32. For example, in the sample pooling container 30, a plurality of pooling chambers 31 may be arranged in an 8x12 orthogonal array, and eight pooling chambers 31 in the y-direction may be grouped into one pooling task group 32, such that a total of 12 groups are formed.

In addition, in the task group grouping step S300, among the sample vessels 20 associated with each pooling chamber 31 belonging to the pooling task group 32, the sample vessels 20 located in the same row or the same column may be grouped into a sample task group 22 with the same processing order. For example, eight samples in the y-direction may be grouped into one sample task group 22.

In addition, the automatic sample pooling system may further include a pool identifier mapping step S210 of generating a pool identifier for the pooled sample located in each pooling chamber 31 of the sample pooling container 30 and mapping the sample identifiers associated with the respective pooling chambers 31 to the pool identifiers.

The plurality of sample vessels 20 may have different sample identifiers. For example, the sample identifier may be a barcode. In addition, the pool identifier may include barcodes associated with the sample pooling container 30 and location information associated with each of the plurality of pooling chambers 31. For example, the location information of the pooling chamber 31 may be expressed as information about the position of the pooling chamber 31 in the sequence, number of between 1 and 98, or coordinate of between (1,1) and (8,12).

In addition, the automatic sample pooling system may further include an error alarm step S410 of, when an error has occurred in the process of transferring the samples from the sample vessels 20 associated with the pooling chamber 31, providing information about at least one of the location of the sample vessel 20 for which the error has occurred and the location of the pooling chamber 31 associated with the sample vessel 20 for which the error has occurred.

In the error alarm step S410, the information about the location of the sample vessel 20 for which the error has occurred may be provided by positioning an indicator on the sample vessel 20. The indicator may include a physical mark that may be visually recognized by the operator. For example, the indicator may be a tip. When an error occurs during a sample aspiration process, the sample transfer mechanism 50 may separate the tip and position the tip within the sample vessel 20.

Alternatively, in the error alarm step S410, the information about the location of the pooling chamber 31 associated with the sample vessel 20 for which the error has occurred may be output on a display unit (not shown) to be provided.

In addition, the automatic sample pooling system may perform the sample pooling step S400 in a state in which the pooling chambers 31 of the sample pooling container 30 contain a lysis buffer.

Alternatively, the pooling chambers 31 of the sample pooling container 30 may be provided to be empty, and a lysis buffer may be injected into the pooling chambers 31 after the transfer of pooled samples is completed.

Alternatively, the automatic sample pooling system may further include a sample lysis container including the pooling chambers 31 containing a lysis buffer. The sample transfer mechanism 50 may aspirate samples from the sample vessels 20, dispense the samples into the pooling chamber 31 of the sample pooling container 30, then aspirate a pooled sample from the pooling chamber 31 of the sample pooling container 30, and dispense the pooled sample into the pooling chamber 31 of the sample lysis container.

A step of transferring the pooled sample from the pooling chamber 31 of the sample pooling container 30 to the pooling chamber 31 of the sample lysis container may be further included, after the sample pooling step S400.

The above description merely explains the idea of the present disclosure and the present disclosure may be changed and modified in various ways without departing from the scope of the present disclosure by those of skill in the art. Accordingly, the embodiments described herein are provided not to limit, but to merely explain the idea of the present disclosure, and the idea of the present disclosure is not limited by the embodiments. The scope of the present disclosure should be construed by the following claims, and all technical ideas within the equivalent scope should be construed as being included in the scope of the present disclosure.

In addition, the terms such as "include," "comprise," or "have" described above mean that the corresponding component may be inherent as long as there is no particular opposing recitation, and thus, it should be interpreted that other components may be further included rather than excluded. All terms used herein, including technical and scientific terms, have the same meaning as commonly understood by those of skill in the art, unless defined otherwise. Terms, such as those defined in commonly used dictionaries, are to be interpreted as having a meaning that is consistent with their meaning in the context of the relevant art and the present disclosure, and are not to be interpreted in an idealized or overly formal sense unless expressly so defined herein.

While specific aspects of the present disclosure have been described in detail above, it will be apparent to those of skill in the art that such specific descriptions are merely preferred embodiments and that the scope of the present disclosure is not limited thereto. Therefore, the substantial scope of the present disclosure should be defined by the appended claims and their equivalents.

**List of Reference Numerals for Major Elements**

| | | | |
|---|---|---|---|
| 10: | Deck, | 11: | Sample mounting portion, |
| 12: | Container mounting portion, | 13: | Waste container, |
| 14: | Barcode reader, | 20: | Sample vessel, |
| 21: | Sample carrier, | 22: | Sample task group, |
| 30: | Sample pooling container, | 31: | Pooling chamber, |
| 32: | Pooling task group, | 40: | Tip container, |
| 50: | Sample transfer mechanism. | | |

## Claims

1. A sample pooling method using an automatic sample pooling system comprising a deck and a plurality of sample transfer mechanisms, the sample pooling method comprising:
a pooling chamber arrangement step of arranging a plurality of pooling chambers in a pooling area of the deck;
a sample vessel arrangement step of arranging a plurality of sample vessels in a sample area of the deck;
a sample association step of associating the sample vessels equal to a preset pool size to each of the pooling chambers, wherein the pool size represents the number of sample vessels to be associated with each of the pooling chambers; and
a sample pooling step of transferring samples from the sample vessels to the associated pooling chamber by using the sample transfer mechanisms, to provide a pooled sample in the pooling chamber where the samples equal to the pool size are combined.

2. The sample pooling method of claim 1, wherein the pool size is selected within a range of 2 to 10.

3. The sample pooling method of claim 1, wherein, in the sample association step, p samples are associated with the pooling chamber, and positions in a processing order are sequentially assigned to the sample vessels associated with the pooling chamber, and
in the sample pooling step, a preset amount of the samples in the sample vessels is transferred to the associated pooling chamber sequentially according to the processing ranks that are assigned to the sample vessels.

4. The sample pooling method of claim 3, further comprising a task group grouping step of grouping the pooling chambers in the pooling area into pooling task groups, wherein each of the pooling task groups comprises the pooling chambers equal to a preset task group size, and the task group size represents the number of pooling chambers included in each of the pooling task groups,
wherein, in the sample pooling step, a process of transferring a preset amount of the sample from each of the sample vessels corresponding to a same processing ranks among the sample vessels associated with each of the pooling chambers in the pooling task group, to each associated pooling chamber is sequentially performed.

5. The sample pooling method of claim 4, wherein, in the pooling area, the plurality of pooling chambers are arranged in an orthogonal array, and
in the task group grouping step, the pooling chambers located in a same row or a same column are grouped into a same pooling task group.

6. The sample pooling method of claim 4, wherein, in the sample area, the plurality of sample vessels are arranged in an orthogonal array, and
in the task group grouping step, the sample vessels located in a same row or a same column among the sample vessels associated with the pooling chamber of the pooling task group are grouped into a sample task group having a same processing order.

7. The sample pooling method of claim 6, wherein the plurality of sample transfer mechanisms comprise the sample transfer mechanisms corresponding to the sample vessels within the sample task group, and
in the sample pooling step, a preset amount of the samples from the sample vessels within the sample task group is transferred to the respective pooling chambers associated with the sample vessels by using the sample transfer mechanisms.

8. The sample pooling method of claim 1, further comprising, before the sample association step, a pool size input step of receiving, as input, the pool size, which is the number of sample vessels to be associated with each of the pooling chambers.

9. The sample pooling method of claim 8, further comprising:
a sample identifier generation step of generating sample identifiers for the samples accommodated in the sample vessels in the sample area, respectively;
a pool identifier generation step of generating pool identifiers for the pooled samples accommodated in the pooling chambers in the pooling area, respectively; and
a pool identifier mapping step of mapping the sample identifiers of the sample vessels associated with each of the pooling chambers, to the pool identifier.

10. The sample pooling method of claim 8, further comprising an error alarm step of, when an error has occurred in a process of transferring the samples from the sample vessels associated with the pooling chamber, providing information about at least one of a location of the sample vessel for which the error has occurred, and a location of the pooling chamber associated with the sample vessel for which the error has occurred.

11. The sample pooling method of claim 10, wherein, in the error alarm step, information about the location of the sample vessel for which the error has occurred is provided by positioning an indicator on the sample vessel.

12. The sample pooling method of claim 10, wherein the sample transfer mechanism comprises a detachable tip for sample aspiration, and
in the error alarm step, the information about the location of the sample vessel for which the error has occurred is provided by moving the sample transfer mechanism to be positioned above the sample vessel for which the error has occurred, and then separating the tip and positioning the tip within the sample vessel.

13. The sample pooling method of claim 1, wherein, in the sample pooling step, the pooled sample is transferred in a state in which the pooling chambers in the pooling area each contain a lysis buffer and/or magnetic beads.

14. The sample pooling method of claim 1, wherein the automatic sample pooling system further comprises a sample lysis area in which a plurality of lysis vessels corresponding to the pooling chambers, respectively, and containing a lysis buffer are arranged, and
the sample pooling method further comprises, after the sample pooling step, a sample lysis step of transferring the pooled sample of each of the pooling chambers in the pooling area, to the corresponding lysis vessel.

15. A computer-readable recording medium comprising instructions for implementing a processor for executing a sample pooling method, the sample pooling method comprising:
a sample association step of associating sample vessels equal to a preset pool size among a plurality of sample vessels in a sample area, to each of a plurality of pooling chambers in a pooling area, wherein the pool size represents the number of sample vessels to be associated with each of the pooling chambers; and
a sample pooling step of transferring samples from the sample vessels to the associated pooling chamber, to provide a pooled sample in the pooling chamber where the samples equal to the pool size are combined.

16. An automatic sample pooling device comprising a computer processor and the computer-readable recording medium of claim 15, which is coupled to the computer processor.

17. A computer program that is stored in a computer-readable recording medium, and implements a processor for executing a sample pooling method, the sample pooling method comprising:
a sample association step of associating sample vessels equal to a preset pool size among a plurality of sample vessels in a sample area, to each of a plurality of pooling chambers in a pooling area, wherein the pool size represents the number of sample vessels to be associated with each of the pooling chambers; and
a sample pooling step of transferring samples from the sample vessels to the associated pooling chamber, to provide a pooled sample in the pooling chamber where the samples equal to the pool size are combined.

18. An automatic sample pooling system comprising:
a deck comprising a sample area in which a plurality of sample vessels are arranged, and a pooling area in which a plurality of pooling chambers are arranged; and
a plurality of sample transfer mechanisms configured to transfer samples from the sample vessels to the pooling chambers,
wherein the number of sample vessels in the sample area is determined by multiplying the number of pooling chambers in the pooling area by a pool size that is selected within a range of 2 to 10.

19. The automatic sample pooling system of claim 18, further comprising a controller configured to associate the sample vessels equal to the pool size, with each of the pooling chambers in the pooling area, and control the sample transfer mechanisms to transfer samples in the sample vessels to the associated pooling chamber, to provide a pooled sample in the pooling chamber where the samples equal to the pool size are combined.

20. The automatic sample pooling system of claim 18, further comprising a controller configured to receive information about the pool size, associate the sample vessels equal to the pool size, with each of the pooling chambers in the pooling area, sequentially assign positions in a processing order to the sample vessels associated with the pooling chamber, and control the sample transfer mechanisms to transfer samples in the sample vessels to the associated pooling chamber according to the assigned processing ranks, to provide a pooled sample in the pooling chamber where the samples equal to the pool size are combined.

21. The automatic sample pooling system of claim 19, wherein the controller is further configured to generate sample identifiers for the samples accommodated in the sample vessels in the sample area, respectively, generate pool identifiers for the pooled samples accommodated in the pooling chambers in the pooling area, respectively, and associate the sample identifiers of the sample vessels associated with each of the pooling chambers, to the pool identifier.

22. The automatic sample pooling system of claim 19, wherein the controller is further configured to, when an error has occurred during a process of transferring the samples from the sample vessels associated with the pooling chamber, receive an error signal from the sample transfer mechanism and provide information about at least one of a location of the sample vessel for which the error has occurred and a location of the pooling chamber associated with the sample vessel for which the error has occurred.

23. The automatic sample pooling system of claim 22, wherein the controller is further configured to output an instruction to position an indicator on the sample vessel for which the error has occurred, to provide the information about the location of the sample vessel for which the error has occurred.

24. The automatic sample pooling system of claim 22, wherein the controller is further configured to output an instruction to display the information about the location of the pooling chamber associated with the sample vessel for which the error has occurred, to provide the information about the location of the pooling chamber associated with the sample vessel for which the error has occurred.

25. The automatic sample pooling system of claim 18, wherein the pooling chambers in the pooling area each contain a lysis buffer and/or magnetic beads.

26. The automatic sample pooling system of claim 18, further comprising a sample lysis area in which a plurality of lysis vessels corresponding to the pooling chambers, respectively, and containing a lysis buffer and/or magnetic beads are arranged.

27. The automatic sample pooling system of claim 18, wherein the pooling area is configured to have mounted thereon the pooling chambers arranged in 8 rows and 12 columns.
